# EUROPEAN PATENT APPLICATION

(11) **EP 4 517 769 A1**
(43) Date of publication of application: **05.03.2025**
(21) Application number: 24194745.6
(22) Date of filing: 15.08.2024
(51) Int. Cl.: G16H 30/20, G16H 40/63

(54) **PROGRAM, INFORMATION PROCESSING DEVICE, AND INFORMATION PROCESSING METHOD**

(30) Priority: 29.08.2023 JP 2023139180
(71) Applicant: Casio Computer Co., Ltd., Tokyo 151-8543 (JP)
(72) Inventor: ISHIHARA, Muneyuki, Tokyo, 205-8555 (JP); OOKI, Takeshi, Tokyo, 205-8555 (JP)
(74) Representative: Plougmann Vingtoft a/s

(57) **Abstract**

Provided is an information processing technology capable of easily displaying a desired medical image in cooperation with an application of a system such as an electronic medical record system.

A program of an application (12) of an image management system that manages a medical image (3) causes a computer to execute: an image acquisition process of acquiring the medical image (3) of a patient identified by identification information based on the identification information of the patient included in a notification from a medical application (11) that displays information of the patient, the medical application (11) being different from the application (12) of the image management system; and an image display process of causing a display device to display the acquired medical image (3) of the patient identified by the identification information.

## Description

### CROSS-REFERENCE TO RELATED APPLICATIONS

This application claims priority to and the benefit of Japanese Patent Application No. 2023-139180 filed on August 29, 2023. The entire specification, claims, and drawings of Japanese Patent Application No. 2023-139180 are incorporated herein by reference.

### Technical Field

The disclosure of the present specification relates to a program, an information processing device, and an information processing method.

### Background Art

An electronic medical record system is a system that electronizes and centrally manages records (medical records) of consultations, prescriptions, and the like, and has been widely used in medical institutions such as hospitals and clinics. Such an electronic medical record system is described in, for example, JP 2018-032110 A. Furthermore, many medical images are handled in medical institutions, and thus, introduction of a medical image management system having functions such as storage, viewing, and management of medical images is also in progress.

### Summary of Invention

### Technical Problem

In medical institutions, a situation in which it is necessary to view a medical image of a patient may occur, for example, in the middle of a consultation performed by a doctor while referring to an electronic medical record of the patient.

However, since the system that manages electronic medical records and the system that manages medical images are separate systems, it is necessary to manually input patient information (for example, a patient ID) displayed on an application of one of the systems into an application of the other system in order to cope with the above situation. Such work of manually inputting the already displayed information leads to a waste of valuable consultation time, which is undesirable. Furthermore, it is desirable to avoid re-input of information in order to suppress the risk of data mix-up due to an input error or the like.

Although the middle of the consultation has been described as an example as above, a situation in which information needs to be re-input due to the fact that applications of a plurality of systems do not cooperate may occur in various scenes without being limited to the middle of the consultation.

In view of the above circumstances, an object of one aspect of the present disclosure is to provide an information processing technology capable of easily displaying a desired medical image in cooperation with an application of a system such as an electronic medical record system.

### Solution to Problem

According to one aspect of the present disclosure, a program of an application of an image management system that manages a medical image causes a computer to execute: an image acquisition process of acquiring the medical image of a patient identified by identification information based on the identification information of the patient included in a notification from a medical application that displays information of the patient, the medical application being different from the application of the image management system; and an image display process of causing a display device to display the acquired medical image of the patient identified by the identification information.

According to one aspect of the present disclosure, an information processing device in which a program of an application of an image management system that manages a medical image is installed includes a control unit, in which the control unit is configured to execute: an image acquisition process of acquiring the medical image of a patient identified by identification information based on the identification information of the patient included in a notification from a medical application that displays information of the patient, the medical application being different from the application of the image management system; and an image display process of causing a display device to display the acquired medical image of the patient identified by the identification information.

According to one aspect of the present disclosure, an information processing method performed by an information processing device in which a program of an application of an image management system that manages a medical image is installed includes: an image acquisition step of acquiring the medical image of a patient identified by identification information based on the identification information of the patient included in a notification from a medical application that displays information of the patient, the medical application being different from the application of the image management system; and an image display step of causing a display device to display the acquired medical image of the patient identified by the identification information.

### Advantageous Effects of Invention

According to the above-described mode, there is provided the information processing technology capable of easily displaying the desired medical image in cooperation with the application of the system such as an electronic medical record system.

### Brief Description of Drawings

Fig. 1 is a diagram illustrating a configuration of an information processing system according to an embodiment of the present disclosure.
Fig. 2 is a diagram illustrating a configuration of applications operating in the information processing system according to the embodiment of the present disclosure.
Fig. 3 is an example of a screen of an image management application displayed on an information processing device according to the embodiment of the present disclosure.
Fig. 4 is a sequence diagram illustrating an example of an application cooperation process performed by the information processing system according to the embodiment of the present disclosure.
Fig. 5 is a continuation of the sequence diagram illustrated in Fig. 4.
Fig. 6 is another example of the screen of the image management application displayed on the information processing device according to the embodiment of the present disclosure.
Fig. 7 is still another example of the screen of the image management application displayed on the information processing device according to the embodiment of the present disclosure.
Fig. 8 is still another example of the screen of the image management application displayed on the information processing device according to the embodiment of the present disclosure.
Fig. 9 is a block diagram for describing an example of a hardware configuration of a computer that achieves the information processing device according to the embodiment of the present disclosure.

### Description of Embodiments

A program, an information processing device, and an information processing method according to an embodiment of the present disclosure will be described in detail with reference to the drawings.

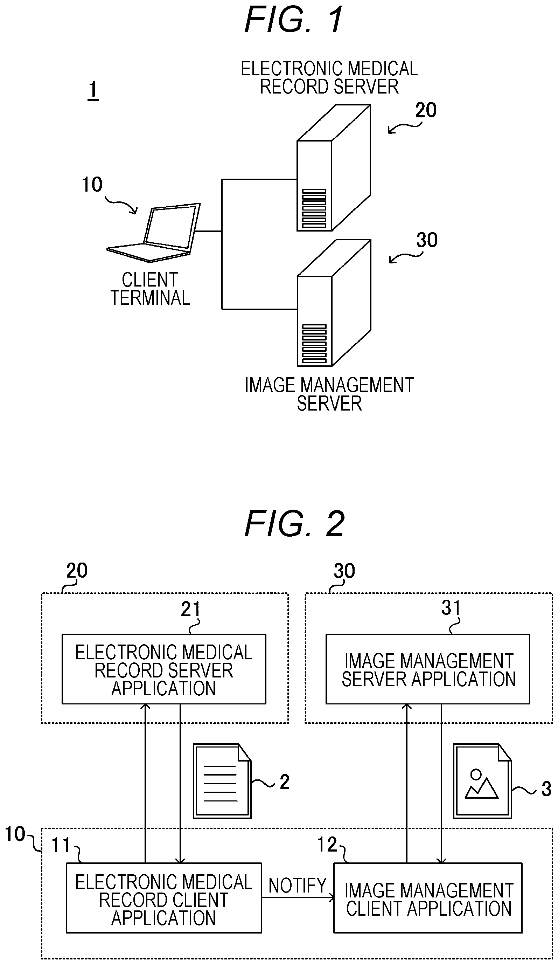

Hereinafter, a configuration of an information processing system 1 will be described with reference to Figs. 1 and 2.

The information processing system 1 is a system used in a medical institution or the like, and includes a client terminal 10, an electronic medical record server 20, and an image management server 30 as illustrated in Fig. 1.

The client terminal 10 is, for example, a device directly operated by a doctor or the like, and is an example of the information processing device of the present disclosure. The client terminal 10 is, for example, any information processing device such as a general-purpose computer, a tablet terminal, or a smartphone.

The electronic medical record server 20 is a server that manages electronized medical record information (hereinafter, referred to as electronic medical records).

The image management server 30 is a server that manages medical images. The medical images managed by the image management server 30 may include images acquired by various devices. For example, a skin image acquired by an imaging device with dermoscopy may be included, and images acquired by a computed tomography (CT) device, a magnetic resonance imaging (MRI) device, and the like may be included. Each image desirably includes, as metadata, identification information for identifying a patient who is a subject, in addition to capturing date and time.

The client terminal 10, the electronic medical record server 20, and the image management server 30 only need to be capable of transmitting and receiving information to and from each other. These devices may be connected via the Internet or may be disposed in a LAN of the same base.

The client terminal 10 and the electronic medical record server 20 constitute an electronic medical record system that handles medical record information (an electronic medical record 2). In the electronic medical record server 20, a server application (an electronic medical record server application 21) of the electronic medical record system operates. Furthermore, a client application (an electronic medical record client application 11) of the electronic medical record system is installed in the client terminal 10. The electronic medical record client application 11 is an example of a medical application. The electronic medical record server application 21 is configured to provide the electronic medical record 2 to the electronic medical record client application 11 in response to a request from the electronic medical record client application 11. The electronic medical record client application 11 displays the electronic medical record 2 acquired from the electronic medical record server application 21 in an application screen displayed on a display of the client terminal 10.

The client terminal 10 and the image management server 30 constitute an image management system that handles medical images. In the image management server 30, a server application (an image management server application 31) of the image management system operates. Furthermore, a client application (an image management client application 12) of the image management system is installed in the client terminal 10. The image management server application 31 is configured to provide a medical image 3 to the image management client application 12 in response to a request from the image management client application 12. The image management client application 12 displays the medical image 3 acquired from the image management server application 31 in an application screen displayed on the display of the client terminal 10.

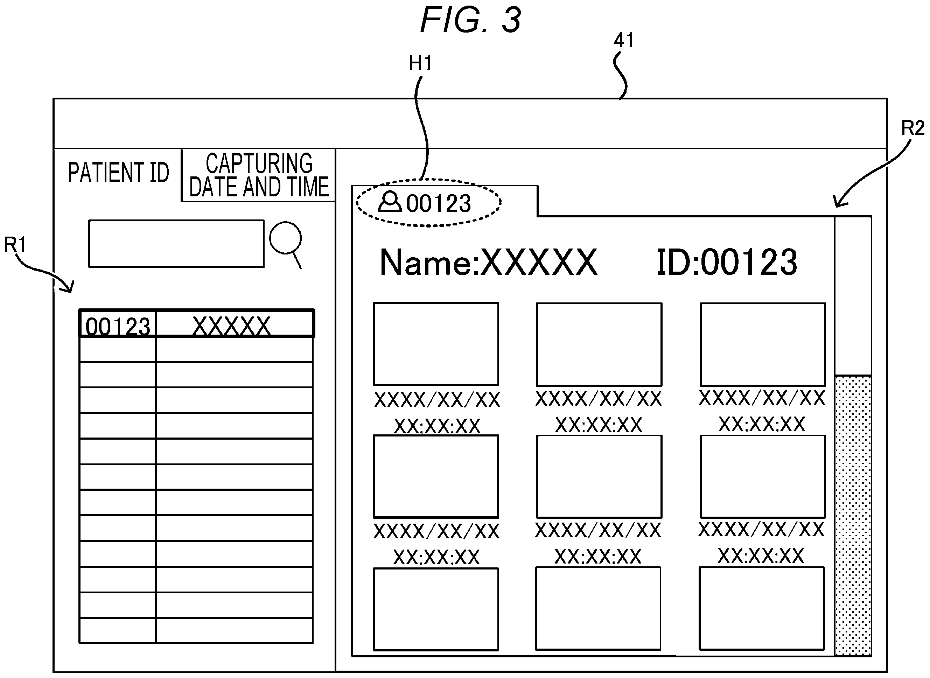

Hereinafter, a configuration of the application screen of the image management client application 12 will be described with reference to Fig. 3.

The application screen of the image management client application 12 includes, for example, a region R1 for designating a search condition and a region R2 for displaying a search result as in an application screen 41 illustrated in Fig. 3.

A search window is provided in the region R1. A patient ID and capturing date and time input to the search window are used as search conditions at the time of searching for a medical image. That is, the image management client application 12 notifies the image management server application 31 of information input to the search window as a search condition, and the image management server application 31 searches for a medical image based on the notified search condition.

In the region R2, a list of medical images searched based on a search condition input to the search window is displayed. More specifically, if search is performed with a search condition designated in the region R1, the image management client application 12 creates a tab including a medical image corresponding to the designated search condition in the region R2, and displays the designated search condition in a header of the tab. If the search is repeated, a new tab is added to the region R2. The image management client application 12 can display a medical image corresponding to a search condition desired by a user by switching an active tab according to a tab selected by the user. Fig. 3 illustrates a state in which a patient ID "00123" is displayed in a header H1 of a tab as a result of search with the patient ID "00123" designated as a search condition.

As described above, the user can search for a medical image under a desired search condition by inputting the search condition to the search window of the image management client application 12, and can refer to the medical image obtained as a result on the application screen 41.

By the way, there is a case where it is desired to search for a medical image by designating information on the electronic medical record client application 11 as a search condition of the image management client application 12 in a case where the electronic medical record client application 11 different from the image management client application 12 is operated on the client terminal 10 as illustrated in Fig. 2.

In such a case, in the related art, two operations on the image management client application 12, that is, (1) an operation of activating the image management client application 12 and (2) an operation of inputting the search condition to a search window of the image management client application 12 with reference to the information on the electronic medical record client application 11, are required.

On the other hand, in the information processing system 1, since the image management client application 12 is configured to process the notification from the electronic medical record client application 11 in advance, the user can display a desired medical image on the image management client application 12 by operating the electronic medical record client application 11.

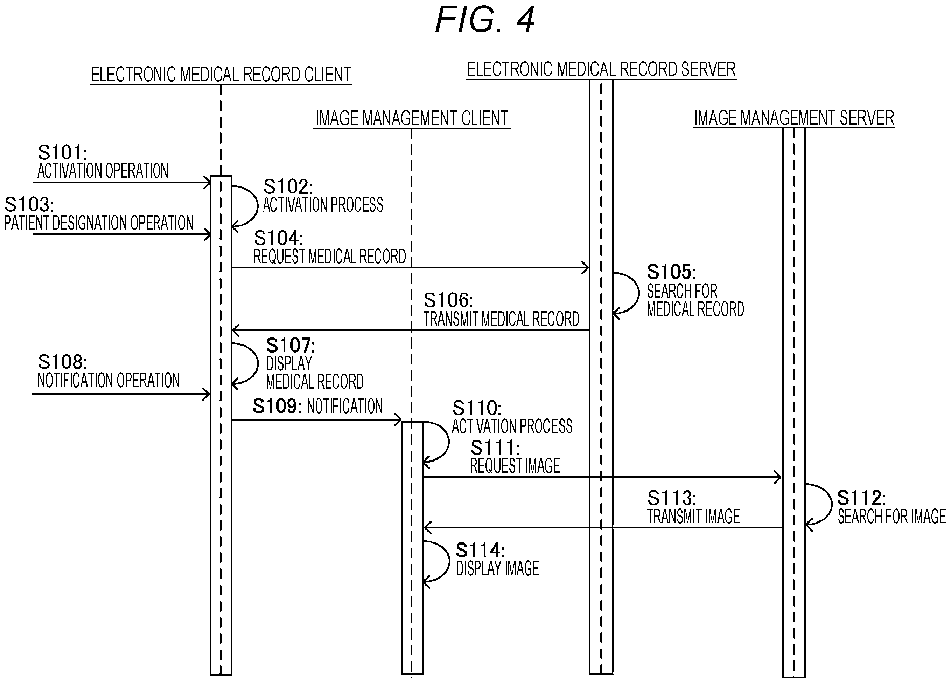

Hereinafter, application cooperation between the electronic medical record client application 11 and the image management client application 12 will be described with reference to Figs. 4 to 8 by exemplifying a case where the user (for example, a doctor) of the client terminal 10 confirms a medical image of a first patient after confirming an electronic medical record of the patient, further confirms a medical image of a second patient after confirming an electronic medical record of the patient, and finally ends the applications.

Here, it is assumed that the electronic medical record server application 21 and the image management server application 31 have been activated on the servers (the electronic medical record server 20 and the image management server 30), and the electronic medical record client application 11 and the image management client application 12 are activated by the user on the client terminal 10 as necessary.

First, in steps S101 to S107 in Fig. 4, the electronic medical record system displays the electronic medical record of the first patient with the patient ID "00123" based on the user's operation.

If the user performs an operation for activating the electronic medical record client application 11 on the client terminal 10 (step S101), a control unit of the client terminal 10 executes a program of the electronic medical record client application 11 to activate the electronic medical record client application 11 (step S102).

Further, if the user performs an operation of designating the first patient, for example, an operation of designating the patient ID "00123", on the electronic medical record client application 11 in order to confirm the electronic medical record of the first patient with the patient ID "00123" (step S103), the electronic medical record client application 11 requests the electronic medical record server application 21 to transmit the electronic medical record of the first patient (step S104).

In response to the request from the electronic medical record client application 11, the electronic medical record server application 21 searches for the electronic medical record of the first patient (patient ID "00123") (step S105), and then transmits the electronic medical record of the patient ID "00123" to the electronic medical record client application 11 (step S 106). The electronic medical record client application 11 having received the electronic medical record of the patient ID "00123" from the electronic medical record server application 21 displays the electronic medical record on the application screen (step S107).

Next, in steps S108 to S 113 in Fig. 4, the electronic medical record client application 11 and the image management client application 12 cooperate with each other based on the user's operation to display the medical image of the first patient with the patient ID "00123" on the image management client application 12.

If the user performs a predetermined operation for notifying the image management client application 12 of the patient ID of the first patient with respect to the electronic medical record client application 11 (step S108), the electronic medical record client application 11 transmits a notification including the patient ID to the image management client application 12 (step S109).

The notification from the electronic medical record client application 11 to the image management client application 12 in step S109 is not particularly limited, but may include a command for executing a program of the image management client application 12, and the patient ID may be designated as a command line argument. Specifically, examples thereof include "program name of image management client application 12.exe/patentid=00123".

If the notification is transmitted, in a state in which the image management client application 12 is not activated, the control unit of the client terminal 10 executes the command included in the notification to execute the program of the image management client application 12, and an activation process of the image management client application 12 is performed (step S110). Then, in the activation process or the subsequent process, the image management client application 12 requests the image management server application 31 to transmit the medical image of the patient identified by the patient ID given as the command line argument (activation argument) (step S111).

In response to the request from the image management client application 12, the image management server application 31 searches for the medical image of the first patient (patient ID "00123") (step S 112), and then transmits the medical image of the patient ID "00123" to the image management client application 12 (step S113). The image management client application 12 having received the medical image of the patient ID "00123" from the image management server application 31 displays the medical image on the application screen 41 illustrated in Fig. 3 (step S114). The application screen 41 is an application screen in which the tab in which patient identification information ("00123") is displayed in the header H1 is made active.

Note that the process in step S111 and the process of receiving the medical image transmitted in step S113 performed by the image management client application 12 are an example of an image acquisition process of acquiring a medical image of a patient identified by identification information based on the identification information (patient ID) of the patient included in a notification from the electronic medical record client application 11. Furthermore, the process in step 5114 performed by the image management client application 12 is an example of an image display process of displaying an acquired medical image of a patient identified by identification information on a display device.

Further, the process in step S 110 performed by the image management client application 12 is an example of the activation process of the image management client application 12 performed if a notification is transmitted before activation of the image management client application 12. Therefore, the process in step S111 and the process of receiving the medical image transmitted in step S 113 performed by the image management client application 12 are an example of the image acquisition process performed during the activation process or subsequent to the activation process, and the process in step S114 is an example of the image display process performed during the activation process or subsequent to the activation process.

As described above, in the information processing system 1 in which the electronic medical record client application 11 and the image management client application 12 cooperate with each other, the image management client application 12 can be automatically activated to display a desired medical image on the application screen of the image management client application 12 by operating the electronic medical record client application 11. Furthermore, since the identification information (patient ID) of the patient is included in the notification from the electronic medical record client application 11 to the image management client application 12, the desired medical image can be reliably displayed, and it is possible to prevent a medical image mismatch caused by an input mistake or the like.

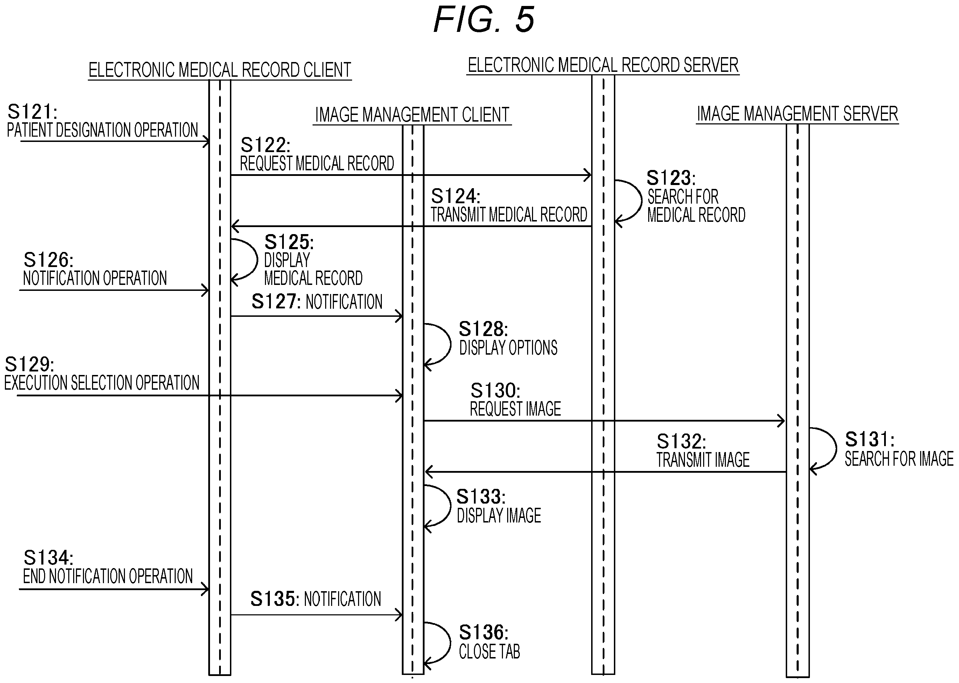

Further, in steps S121 to S125 in Fig. 5, the electronic medical record system displays the electronic medical record of the second patient with a patient ID "00254" based on the user's operation.

If the user performs an operation of designating the second patient, for example, an operation of designating the patient ID "00254", on the electronic medical record client application 11 in order to confirm the electronic medical record of the second patient with the patient ID "00254" (Step S121), the electronic medical record client application 11 requests the electronic medical record server application 21 to transmit the electronic medical record of the second patient (Step S122).

In response to the request from the electronic medical record client application 11, the electronic medical record server application 21 searches for the electronic medical record of the second patient (patient ID "00254") (step S123), and then transmits the electronic medical record of the patient ID "00254" to the electronic medical record client application 11 (step S124). The electronic medical record client application 11 having received the electronic medical record of the patient ID "00254" from the electronic medical record server application 21 displays the electronic medical record on the application screen (step S125).

Next, in steps S126 to S133 in Fig. 5, the electronic medical record client application 11 and the image management client application 12 cooperate with each other based on the user's operation to display the medical image of the second patient with the patient ID "00254" on the image management client application 12.

If the user performs a predetermined operation for notifying the image management client application 12 of the patient ID of the second patient on the electronic medical record client application 11 (step S126), the electronic medical record client application 11 transmits a notification including the patient ID to the image management client application 12 (step S127).

The notification from the electronic medical record client application 11 to the image management client application 12 in step S127 is similar to that in step S109 except for a value of the patient ID.

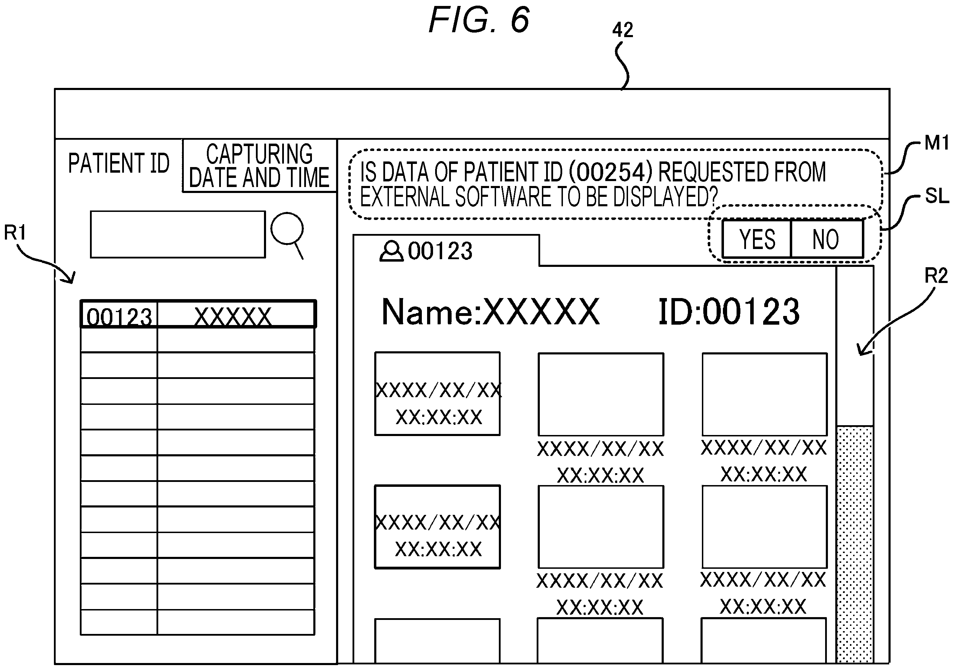

If the notification is transmitted from the electronic medical record client application 11 to the image management client application 12, the activation process of the image management client application 12 is not performed in a state in which the image management client application 12 is activated. Instead, the already activated image management client application 12 updates the application screen and displays options related to the notification on the image management client application 12 (step S128). Specifically, for example, as illustrated in Fig. 6, the image management client application 12 displays an option display SL as to whether or not to display a medical image of a patient ID notified from the electronic medical record client application 11 on an application screen 42 together with a message M1 for describing the options.

Thereafter, if the user performs an operation of selecting "Yes" in the option display SL, that is, selection of execution (an execution selection operation) (step S129), the image management client application 12 detects the selection and requests the image management server application 31 to transmit the medical image of the patient identified by the patient ID given by a command line argument (activation argument) in step S127 (step S130). If the user performs an operation of selecting "NO" in the option display SL, that is, selection of non-execution, the message M1 and the option display SL disappear from the application screen 42, and the application screen is updated to the screen before the notification arrives, that is, the application screen 41.

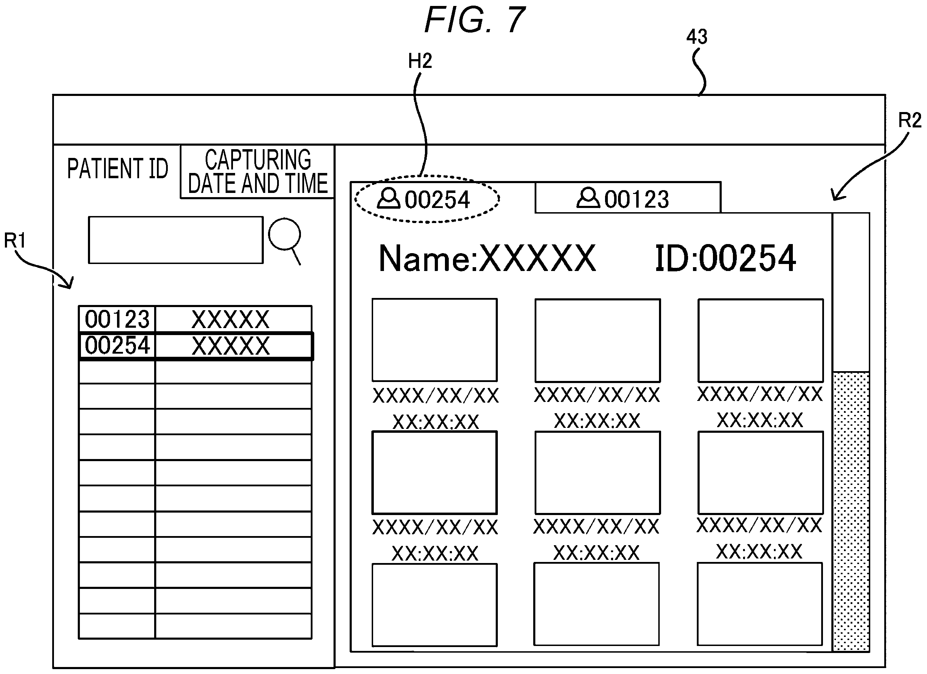

In response to the request from the image management client application 12, the image management server application 31 searches for the medical image of the second patient (patient ID "00254") (step S131), and then transmits the medical image of the patient ID "00254" to the image management client application 12 (step S132). The image management client application 12 having received the medical image of the patient ID "00254" from the image management server application 31 displays the medical image on an application screen 43 illustrated in Fig. 7 (step S133). Here, as illustrated in Fig. 7, the image management client application 12 newly generates a tab including the medical image of the patient ID "00254" and makes the newly created tab active to display the medical image of the patient ID "00254" on the application screen 43. At this time, the patient ID "00254" is displayed in a header H2 of the tab.

Note that the process in step S128 performed by the image management client application 12 is an example of an option display process of causing the display device to display options as to whether or not to execute the image acquisition process and the image display process, which are performed if a notification is transmitted after activation of the image management client application 12. Furthermore, the process in step S130 performed by the image management client application 12 and the process of receiving the medical image transmitted in step S 132 are an example of an image acquisition process performed if selection of execution among the options is detected after the option display process, and the process in step S133 is an example of an image display process performed if the selection of execution among the options is detected after the option display process.

As described above, in the information processing system 1 in which the electronic medical record client application 11 and the image management client application 12 cooperate with each other, the electronic medical record client application 11 can be operated to display a desired medical image on the application screen of the image management client application 12 that has been already activated. Furthermore, an operation timing in the electronic medical record client application 11 and a display timing in the image management client application 12 can be made different by giving an opportunity to select whether or not to display a medical image before the medical image is displayed on the application screen of the image management client application 12, and the medical image can be displayed on the image management client application 12 at a timing desired by the user. Furthermore, the latest search result can be displayed on the application screen by creating a new tab made active on the application screen and displaying the medical image in the tab, and a medical image previously searched for under a different search condition can also be continuously displayed by switching the tab.

Next, in steps S134 to S136 in Fig. 5, the electronic medical record client application 11 and the image management client application 12 cooperate with each other based on the user's operation to end the display of the medical image on the image management client application 12.

If the user performs a predetermined operation for ending the display of the medical image displayed in the image management client application 12 on the electronic medical record client application 11 (step S134), the electronic medical record client application 11 transmits a notification to the image management client application 12 (step S135).

The notification from the electronic medical record client application 11 to the image management client application 12 in step S135 is not particularly limited, but may include a command for executing the program of the image management client application 12 and give an instruction to close the tabs as a command line argument. Specifically, examples thereof include "program name of the image management client application 12.exe/date=closealltabs".

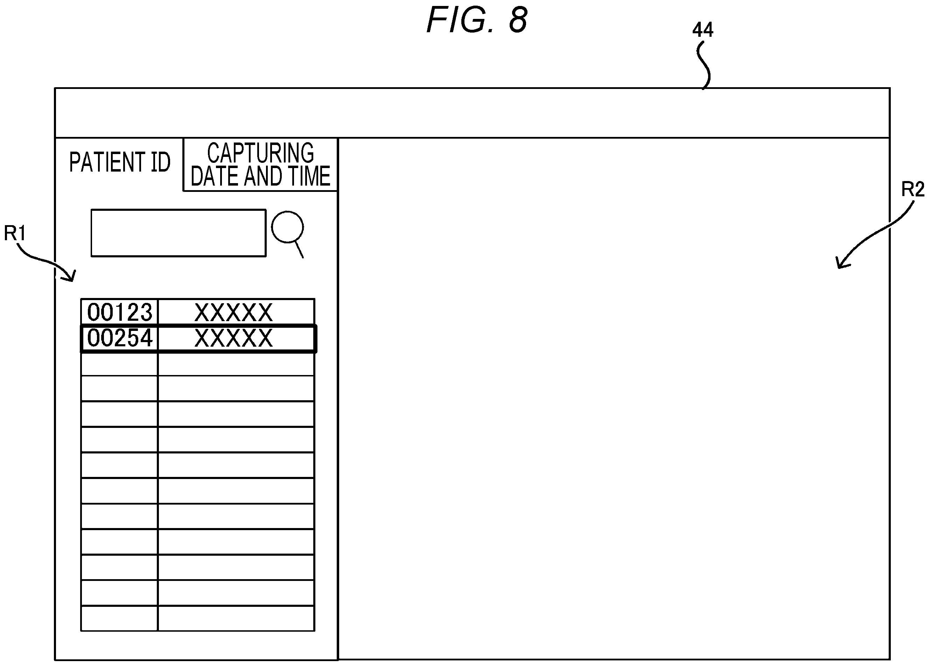

If the notification is transmitted from the electronic medical record client application 11 to the image management client application 12, the image management client application 12 detects the instruction to close the tabs based on the command line argument, and executes a process of closing all the tabs constituting the application screen (step S136). As a result, the application screen is updated from the application screen 43 illustrated in Fig. 7 to an application screen 44 having no tab illustrated in Fig. 8.

Note that the process in step S136 performed by the image management client application 12 is an example of a process of closing all tabs constituting an application screen of the image management client application 12 based on information instructing to close the tabs included in a notification transmitted from the electronic medical record client application 11.

As described above, in the information processing system 1 in which the electronic medical record client application 11 and the image management client application 12 cooperate with each other, it is possible to close a tab already displayed on the application screen of the image management client application 12, that is, to end the display of the medical image by operating the electronic medical record client application 11. As a result, for example, it is possible to avoid a medical image mismatch or the like caused by display of a medical image of a patient whose consultation has ended on the image management client application 12.

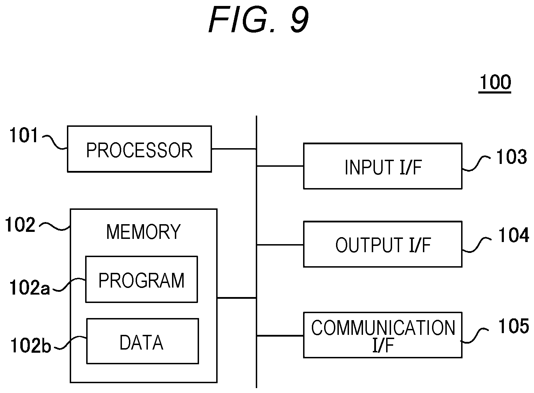

As illustrated in Fig. 9, a computer 100 that implements the client terminal 10 which is the information processing device includes a processor 101, a memory 102, an input interface 103, an output interface 104, and a communication interface 105.

The processor 101 is the control unit of the client terminal 10 and is not particularly limited, but may be, for example, a central processing unit (CPU) or a graphics processing unit (GPU). Furthermore, the processor may include a hardware circuit such as a field-programmable gate array (FPGA) or an application specific integrated circuit (ASIC).

The memory 102 is not particularly limited, but may be a semiconductor memory such as a random access memory (RAM), a read only memory (ROM), or a solid state drive (SSD), a magnetic storage device such as a hard disk drive (HDD), or an optical storage device. The memory 102 stores a program 102a and various types of data 102b for implementing the cooperation between the electronic medical record client application 11 and the image management client application 12 described above. Note that the program 102a includes, for example, the program of the electronic medical record client application 11 and the program of the image management client application 12.

The input interface 103 is connected to a touch panel, a keyboard, or the like. Furthermore, the input interface 103 may be connected to a voice input device such as a microphone. The output interface 104 is connected to a display device or the like. The display device may be the display device included in the client terminal 10. The communication interface 105 transmits and receives data to and from, for example, the electronic medical record server 20 and the image management server 30 via a network.

The above-described embodiment has been given as a specific example to facilitate understanding of the invention, and the present disclosure is not limited to the above-described embodiment, and needs to be understood as including various modifications and alternative forms of the above-described embodiment. For example, it will be understood that the above-described embodiment can be embodied by modifying the components without departing from the spirit thereof. Furthermore, it will be understood that various embodiments can be implemented by appropriately combining a plurality of components disclosed in the above-described embodiment. Moreover, a person skilled in the art will understand that various embodiments can be implemented by deleting some components from all the components indicated in the embodiments or adding some components to the components indicated in the embodiments. That is, the above-described program, information processing method, and information processing device described above can be variously modified and changed without departing from the scope of the claims.

In the above-described embodiment, an example in which only one patient ID is included in a notification transmitted from the electronic medical record client application 11 to the image management client application 12 has been described, but a plurality of patient IDs may be included. Specifically, examples thereof include "program name of image management client application 12.exe/patentid=00123/patentid=254/...". The image management client application 12 may generate a plurality of tabs respectively corresponding to the plurality of patient IDs included in the notification on an application screen and make one of them to be displayed. Note that the tabs include medical images of patients identified by the patients ID, respectively. As a result, it is possible to search for medical images of many patients at a time and display the medical images on the image management client application 12.

Furthermore, in the above-described embodiment, an example in which a medical image is searched with a patient ID has been described, but a search condition is not limited to the patient ID. A capturing date may be included in a notification transmitted from the electronic medical record client application 11 to the image management client application 12. Specifically, examples thereof include "program name.exe/date=yyyymmdd of image management client application 12". The image management client application 12 having received the notification may request the image management server application 31 to transmit a medical image captured on the capturing date and display the acquired medical image. Furthermore, the notification may include both a patient ID and a capturing date, and the image management client application 12 may display a medical image searched using both the patient ID and the capturing date as search conditions.

Furthermore, in the above-described embodiment, an example in which the client application and the server application are installed on different devices has been described, but the client application and the server application may be installed on the same device. For example, the image management server application 31 may be installed in the client terminal 10 in addition to the image management client application 12, the client terminal 10 may operate as the image management server 30, and the client terminal 10 may perform a search process of searching for a medical image of a patient ID notified from the electronic medical record client application 11.

Furthermore, the electronic medical record server application 21 and the image management server application 31 may be general-purpose database server applications, respectively, and the electronic medical record server application 21 and the image management server application 31 may be the same application. The electronic medical record client application 11 may acquire a desired electronic medical record stored in a database server via the general-purpose database server application, and the image management client application 12 may acquire a desired medical image stored in a database server via the general-purpose database server application.

## Claims

1. A program of an application (12) of an image management system that manages a medical image (3), the program **characterized by** causing a computer to execute:
an image acquisition process of acquiring the medical image (3) of a patient identified by identification information based on the identification information of the patient included in a notification from a medical application (11) that displays information of the patient, the medical application (11) being different from the application (12) of the image management system; and
an image display process of causing a display device to display the acquired medical image (3) of the patient identified by the identification information.

2. The program according to claim 1, **characterized by** causing the computer to further execute
an activation process of the application (12) if the notification is transmitted before activation of the application (12), **characterized in that**
the computer is caused to execute the image acquisition process and the image display process during the activation process or subsequent to the activation process.

3. The program according to claim 1 or 2, **characterized by** causing the computer to further execute
an option display process of displaying, on the display device, options as to whether or not to execute the image acquisition process and the image display process if the notification is transmitted after activation of the application (12), **characterized in that**
the computer is caused to execute the image acquisition process and the image display process if selection of execution among the options is detected after the option display process.

4. The program according to claim 1, **characterized in that**
the image display process includes a process of causing the display device to display an application screen in which a tab is made active, the tab including the medical image (3) of the patient and a header on which the identification information of the patient is displayed.

5. The program according to claim 4, **characterized in that**
the image display process includes a process of causing the display device to display an application screen including a plurality of tabs corresponding to a plurality of pieces of identification information in a case where the notification includes the plurality of pieces of identification information, the application screen having any one of the plurality of tabs made active.

6. The program according to claim 4 or 5, **characterized by** causing the computer to further execute
a process of closing all tabs included in the application screen based on information instructing to close the tabs included in the notification transmitted from the medical application (11).

7. An information processing device (10) in which a program of an application (12) of an image management system that manages a medical image (3) is installed, the information processing device (10) comprising
a control unit (101),
**characterized in that** the control unit (101) is configured to execute:
an image acquisition process of acquiring the medical image (3) of a patient identified by identification information based on the identification information of the patient included in a notification from a medical application (11) that displays information of the patient, the medical application (11) being different from the application (12) of the image management system; and
an image display process of causing a display device to display the acquired medical image (3) of the patient identified by the identification information.

8. An information processing method performed by an information processing device (10) in which a program of an application (12) of an image management system that manages a medical image (3) is installed, the information processing method **characterized by** comprising:
an image acquisition step of acquiring the medical image (3) of a patient identified by identification information based on the identification information of the patient included in a notification from a medical application (11) that displays information of the patient, the medical application (11) being different from the application (12) of the image management system; and
an image display step of causing a display device to display the acquired medical image (3) of the patient identified by the identification information.
